# EUROPEAN PATENT APPLICATION

(11) **EP 4 079 945 A1**
(43) Date of publication of application: **26.10.2022**
(21) Application number: 20903962.7
(22) Date of filing: 18.12.2020
(51) Int. Cl.: C40B 50/06, C40B 40/08, C12N 15/10, C12N 15/11

(54) **METHOD FOR CONSTRUCTING A GENE MUTATION LIBRARY**

(30) Priority: 19.12.2019 CN 201911316464
(71) Applicant: Nanjing GenScript Biotech Co., Ltd., Nanjing, Jiangsu 211100 (CN)
(72) Inventor: DAI, Xiaohui, Nanjing, Jiangsu 211100 (CN); LI, Yifan, Nanjing, Jiangsu 211100 (CN); WU, Cheng-Hsien, Nanjing, Jiangsu 211100 (CN)
(74) Representative: Pharma Patents International AG
(86) International application number: PCT/CN2020/137623
(87) International publication number: WO 2021/121391

(57) **Abstract**

Provided is a large-storage capacity gene mutation library construction method, capable of synthesizing relatively few oligomer sequences, then assembling same to create a large-storage capacity gene mutation library.

## Description

### Technical Field

The present invention relates to the field of genetic engineering, and in particular to the construction of a large-storage capacity gene mutation library. In the present invention, a large-storage capacity gene mutation library is created by synthesizing relatively few oligomer sequences and then assembling same.

### Background Art

*In vitro* molecular optimization can be significantly effectively used to generate improved or novel mutant proteins, identify regulatory sequences, and probe critical residues for structure and function. The construction of a synthetic library using the method of *in vitro* molecular optimization is a very effective way to systematically study the property, regulation and function of a protein. By using the high-throughput semiconductor precise primer pool to synthesize all the primers theoretically required for the construction of the gene mutation library, according to different downstream expression systems, codon optimization can be performed and different codon distribution rate at each mutation position can be set to fundamentally eliminate the problem of introducing unexpected codons or stop codons. The library only contains the required mutants to save time and effort for subsequent screening. However, at present, the number of oligomers synthesized by the high-throughput semiconductor precise primer pool is limited, and the cost for synthesizing a large-storage capacity library (10⁵-10¹⁰) by semiconductor chips is very high.

### References:

Kitzman J O, Starita L M, Lo R S et al., Massively parallel single-amino-acid mutagenesis[J]. Nature Methods, 2015, 12(3):203-206.
Kosuri S, Eroshenko N, Leproust E M et al., Scalable gene synthesis by selective amplification of DNA pools from high-fidelity microchips [J]. Nature Biotechnology, 2010, 28(12): 1295-1299.

### Summary of the Invention

In view of this, invented is a large-storage capacity library construction method, capable of synthesizing relatively few oligomer sequences, then assembling same to obtain a large-storage capacity gene mutation library, which greatly reduces the cost for large-capacity library construction.

Specifically, the present invention relates to the following aspects:
a first aspect of the present invention relates to a method for constructing a large-storage capacity gene mutation library, which comprises:
(1) designing and synthesizing two or more oligomer pools having a mutant nucleotide and a restriction site of a restriction endonuclease according to a nucleotide sequence encoding an amino acid sequence which requires library construction, wherein after digested, two adjacent oligomer pools designed produce same sticky ends;
(2) amplifying the oligomer pools;
(3) assembling the oligomer pools in the reaction system to obtain an assembled oligomer pool;
and (4) amplifying the assembled oligomer pool to obtain the large-storage capacity gene mutation library.

In one embodiment, the step (2) in the method of the present invention comprises: performing PCR amplification on each oligomer pool by respectively using each oligomer pool as a template, using the forward primer and reverse primer designed according to the sequence of each oligomer pool as a primer pair and using a high-fidelity DNA polymerase, to obtain amplified each oligomer pool. Preferably, the PCR amplification system further comprises bovine serum albumin.

In one embodiment, the step (3) in the method of the present invention comprises: adding the amplified each oligomer pool, simultaneously adding the restriction endonuclease and a DNA ligase, and assembling the amplified each oligomer pool by using a restriction-ligation method to obtain the assembled oligomer pool.

In one embodiment, the step (4) in the method of the present invention comprises: performing PCR amplification on the assembled oligomer pool by using the assembled oligomer pool as a template, using a forward primer of a first oligomer pool and a reverse primer of a last oligomer pool as a primer pair and using the high-fidelity DNA polymerase to perform PCR, to obtain the large-storage capacity gene mutation library.

In one embodiment, the storage capacity of the gene mutation library of the present invention is up to 10⁵, preferably up to 10⁶, more preferably up to 10⁷, more preferably up to 10⁸, more preferably up to 10⁹, and more preferably up to 10¹⁰.

In one embodiment, the restriction endonuclease used in the present invention is an IIS restriction endonuclease, and after digested, the adjacent two oligomer pools produce same sticky ends. The type IIS restriction endonuclease is selected from one or more of *Acu*I*, Alw*I*, Bbs*I*, Bbv*I*, Bcc*I*, Bce*AI*, Bci*VI*, Bfu*AI*, Bmr*I*, Bpm*I*, Bpu*EI*, Bsa*I*, Bse*RI*, Bsg*I*, Bsm*AI*, Bsm*BI*, Bsm*FI*, Bsp*MI*, Bsp*QI*, Bsr*DI*, Btg*ZI*, Bts*CI*, Bts*I*, Earl, Eci*I*, Eco*P15I, *Fau*I*, Fok*I*, Hga*I*, Hph*I*, HpyAV, Mbo*II*, Mme*I*, Mnl*I*, Ple*I*, Sap*I and *Sfa*NI*.* Preferably, the type IIS restriction endonuclease is *Bsa*I*.*

In one embodiment, the step (1) in the method of the present invention comprises the following:
(i) identifying the sticky ends in the coding nucleotide sequence, and dividing the sequence into two or more fragments corresponding to the two or more oligomer pools according to the 3' ends of the sticky ends,
(ii) if the sequence is divided into two fragments, then

sequentially introducing a reverse complement sequence of a recognition sequence of the restriction endonuclease and a specific sequence 1 at 3' end after a sticky end of a first fragment, to obtain an oligomer pool 1, and sequentially introducing a sticky end, a recognition sequence of the restriction endonuclease and a specific sequence 2 at 5' end of a second fragment, to obtain an oligomer pool 2;
or if the sequence is divided into n fragments, wherein n is a positive integer greater than or equal to 3, then:
   sequentially introducing a reverse complement sequence of a recognition sequence of the restriction endonuclease and a specific sequence 1 at 3' end after a sticky end of a first fragment, to obtain an oligomer pool 1; and sequentially introducing a sticky end, a recognition sequence of the restriction endonuclease and a specific sequence 2 at 5' end of a second fragment, sequentially introducing a reverse complement sequence of a recognition sequence of the restriction endonuclease and a specific sequence 3 at 3' end after a sticky end of a second fragment, to obtain an oligomer pool 2; and so on, sequentially introducing a sticky end, a recognition sequence of the restriction endonuclease and a specific sequence 2n-2 at 5' end of a n^{th} fragment and sequentially introducing a reverse complement sequence of a recognition sequence of the restriction endonuclease and a specific sequence 2n-1 at 3' end after a sticky end of a n^{th} fragment, to obtain an oligomer pool n.

In one embodiment, the specific sequence 1-the specific sequence 2n-1 can be random sequences that are not homologous to the original coding nucleotide sequence.

In one embodiment, the sticky end is a single sticky end or a degenerate sticky end. In one embodiment, the sticky end is a single sticky end, and the number of the oligomer pools is 2-6, preferably 2. In another embodiment, wherein the sticky end is a degenerate sticky end, and the number of the oligomer pools is 2. In one embodiment, the GC content of the sticky end is 50%-75%. In one embodiment, the sticky end does not contain a palindromic structure.

In one embodiment, the restriction-ligation method of step (3) in the method of the present invention is Golden Gate cloning.

In one embodiment, the method of the present invention further comprises: (5) recovering and/or purifying the gene mutation library product obtained in step (4) to obtain a final library product. Preferably, the gene mutation library product obtained in step (4) is recovered and/or purified by gel electrophoresis.

In one embodiment, the method of the present invention further comprises: (6) sequencing the final library product obtained in step (5) to verify sequence distribution and/or detect amino acid distribution. Preferably, the sequencing is Sanger sequencing and/or NGS sequencing.

In one embodiment, the number of the mutant nucleotide in each oligomer pool synthesized in step (1) is 1-108, preferably 1-21. In one embodiment, the mutant nucleotide in each oligomer pool synthesized in step (1) of the method of the present invention encodes 1-36, preferably 1-7 mutant amino acids. In one embodiment, the mutant amino acids encoded by the mutant nucleotide in each oligomer pool synthesized in step (1) are either adjacent or non-adjacent.

In one embodiment, the high-fidelity DNA polymerase used in the method of the present invention is selected from one or more of Phusion DNA polymerase, Q5 polymerase and primerSTAR polymerase. In a preferred embodiment, the employed high-fidelity DNA polymerase is Phusion DNA polymerase.

A second aspect of the present invention relates to use the method according to the present invention to construct a gene mutation library.

A third aspect of the present invention relates to the use of the gene mutation library constructed by the method according to the present invention in screening proteins or polypeptides.

A fourth aspect of the present invention relates to a method for analyzing the relationship between an amino acid mutation in a protein and the property, regulation and/or function of the protein, comprising the following steps:
(1) using the method according to the present invention to construct a gene mutation library;
(2) comparing the property, regulation and/or function of the protein encoded by a mutant gene in the constructed gene mutation library with that of a unmutated protein; and
(3) analyzing the relationship between the amino acid mutation in the protein and the property, regulation and/or function of the protein.

### The technical problems to be solved by the present invention and the technical effects achieved by the present invention

The mutation library is very important for protein engineering and antibody drug engineering. A lot of time, energy and money for subsequent screening can be saved by using the method of the present invention to construct the mutation library. At present, the number of oligomers synthesized by the prior art (comprising the high-throughput semiconductor precision synthesis of oligomer pools) is limited, and it is very difficult or very expensive to synthesize a large-storage capacity (the storage capacity is more than 10⁵) gene mutation library. Disclosed in the present invention is a large-storage capacity library construction method, capable of synthesizing relatively few oligomer sequences, then assembling same to create a large-storage capacity gene mutation library. The storage capacity can be up to 10⁷-10¹⁰, which greatly saves the cost of constructing a large-storage capacity library, and solves the problem of high cost of synthesizing a large-storage capacity library.

### Brief Description of The Drawings

The present invention will be more fully understood from the following detailed description taken in conjunction with the accompanying drawings.
Fig. 1 is the interface of Library generate procedure for designing an oligomer pool;
Fig. 2 is an electropherogram of amplification products of oligomer pool 1 and pool 2 having a single sticky end (lane 1: DL3000; lane 2: oligomer pool-1; lane 3: oligomer pool -2);
Fig. 3 is a peak map of an oligomer pool after a single sticky end is assembled;
Fig. 4 is an electropherogram of an amplification product of an oligomer pool after a single sticky end is assembled (lane 1: amplified oligomer pool; lane 2: DL3000);
Fig. 5 is a Sanger sequencing result of the library sequence obtained by assembling a single sticky end;
Fig. 6 is a sequence distribution diagram of NGS sequencing of the library sequence obtained by assembling a single sticky end;
Fig. 7 is an amino acid distribution of NGS sequencing of the library sequence obtained by assembling a single sticky end;
Fig. 8 is an electropherogram of an amplification product of an oligomer pool after a degenerate sticky end is assembled (lane 1: DL3000; lane 2: amplified oligomer pool);
Fig. 9 is a Sanger sequencing result of the library sequence obtained by assembling a degenerate sticky end;
Fig. 10 is a sequence distribution diagram of NGS sequencing of the library sequence obtained by assembling a degenerate sticky end;
Fig. 11 is an amino acid distribution of NGS sequencing of the library sequence obtained by assembling a degenerate sticky end.

### Detailed Description of Embodiments

As used in the present description and appended claims, the singular forms "a," "an," and "the" include plural referents, unless the context clearly dictates otherwise. Therefore, for example, reference to "a molecule" optionally includes a combination of two or more of such molecules, and the like.

As used herein, the term "about" refers to a conventional error range of the corresponding numerical value easily known by those skilled in the art. Reference herein to "about" a certain value or a parameter includes (and describes) the embodiment involving the value or parameter itself.

It is understood that the aspects and embodiments of the present invention described herein include aspects and embodiments involving "comprise", "consist of', and "essentially consist of".

The term "gene mutation library" refers to a combination of a large amount of DNA variant sequences, which is a product of gene synthesis, gene mutation and/or directed evolution study. The gene mutation library has been increasingly used in research fields, such as high-throughput drug target screening, directed evolution by protein engineering, synthesizing diversity antibody libraries to screen variant antibodies with high-affinity and specificity, *etc.* The construction of a gene mutation library comprises using techniques such as site-directed mutagenesis and directed evolution to simulate the natural selection process, thereby changing the amino acid sequence of the original protein and obtain a mutant protein with specific function. Gene mutation libraries comprise: alanine scanning library, degenerate mutation library, trimer library, site-directed saturation mutation library, random mutation library, *etc.*

The term "storage capacity of the gene mutation library" refers to the capacity size of the gene mutation library, that is the number of DNA variant sequences contained in the gene mutation library. In other words, the larger the storage capacity of the gene mutation library, the greater the number of DNA variant sequences contained in the library.

The term "oligomer "generally refers to a polymer composed of relatively few repeating units, and the relative molecular mass thereof is between small molecule and macromolecule. The oligomer of the present invention refers to a nucleotide oligomer containing a certain number of nucleotides, and can be used interchangeably with oligonucleotide. The number of nucleotides contained in the oligomer of the present invention can be 2-200, 3-150, 4-100, 4-50 and 4-30. The term "oligomer pool" in the present invention refers to a mixture containing a plurality of different oligomers.

The term "mutant nucleotide" refers to a nucleotide that is altered at one or more (e.g., several) positions in a polynucleotide or oligonucleotide sequence. The alteration is selected from substitution, deletion, and insertion. Substitution means replacing a nucleotide occupying a position with a different nucleotide; deletion means removing a nucleotide occupying a position; and insertion means adding a nucleotide at a position that is adjacent to and immediately following a nucleotide occupying a position. Similarly, the term "mutant amino acid" refers to an amino acid that is altered at one or more (e.g., several) positions in a protein or polypeptide sequence. The alteration is selected from substitution, deletion, and insertion. Substitution means replacing an amino acid occupying a position with a different amino acid; deletion means removing an amino acid occupying a position; and insertion means adding an amino acid at a position that is adjacent to and immediately following an amino acid occupying a position.

The term "restriction endonuclease" refers to a class of active proteins that recognize a specific nucleotide sequence and cleave the phosphodiester bond between two nucleotides at specific locations in each strand. Restriction endonucleases are widely distributed, and almost all bacterial genera and species have at least one restriction endonuclease. Common restriction sites include, but are not limited to: *Age*I*, BamHl, Bgl*II*, Eco*RI*, Eco*RV*, Fse*I*, Hind*III*, Mau*BI*, Mlu*I*, NheI, Not*I*, Pac*I*, Pme*I*, Pst*I*, Sac*I*, Sac*II*, Sal*I*, Sma*I*, Spe*I*, Sfi*I*, etc.* Introducing a restriction site into a DNA sequence may facilitate molecular biological manipulation on the DNA sequence.

Restriction endonucleases can be divided into the following types according to the structural/functional property, size, required cofactor, and mode of action thereof: type I restriction endonuclease, type II restriction endonuclease and type III restriction endonuclease. Type I restriction endonuclease has a relatively high molecular weight, and requires S-adenosyl-L-methionine, ATP, *etc.* in addition to Mg²⁺ in the reaction process. It can catalyze the methylation of host DNA (i.e., modification) and also catalyze the hydrolysis of unmethylated DNA (i.e., recognition and enzymatic digestion). Usually, the restriction site of type I restriction endonuclease is thousands of bases away from the recognition sequence. Type II restriction endonuclease usually has a relatively small molecular weight and requires only Mg²⁺ for the reaction. It only catalyzes the hydrolysis of unmethylated DNA and has a specific restriction site on the recognition sequence (a specific short palindromic sequence). Therefore, it is widely used in genetic engineering. Type III restriction endonuclease is similar to type I restriction endonuclease, and has the functions of modification, recognition and enzymatic digestion. However, it recognizes short asymmetric sequences, and the distance between the restriction site and the recognition sequence is approximately 20-30 base pairs. In addition, there is a type IIS restriction endonuclease. It recognizes continuous asymmetric sequences, and the distance between the restriction site and the recognition sequence is approximately 1-6 base pairs. The size of the IIS restriction endonuclease is medium and about 400-650 amino acids. It is generally admitted that the type IIS restriction endonuclease binds to DNA mainly in the form of monomer, however combines with adjacent enzyme to form a dimer to cooperatively cut DNA strands. Therefore, some type IIS enzymes may be more active in cleaving DNA molecules with multiple recognition sequences. Common type IIS restriction endonucleases include, but are not limited to *Acu*I*, Alw*I*, Bbs*I*, Bbv*I*, Bcc*I*, Bce*AI*, Bci*VI*, Bfu*AI*, Bmr*I*, Bpm*I*, Bpu*EI*, Bsa*I*, Bse*RI*, Bsg*I*, Bsm*AI*, Bsm*BI*, BsmFI, Bsp*MI*, Bsp*QI*, Bsr*DI*, Btg*ZI*, Bts*CI*, Bts*I*, Ear*I*, Eci*I*, Eco*P15I*, Fau*I*, Fok*I*, Hga*I*, Hph*I*, Hpy*AV*, Mbo*II*, MmeI, Mnl*I*, Ple*I*, Sap*I*, Sfa*NI*, etc.*

The term "DNA ligase" refers to an enzyme that can form a phosphodiester bond between the terminal 3'-OH of one DNA strand and the terminal 5'-P of another DNA strand, thereby ligating two adjacent DNA strands. The catalysis of a ligase requires consumption of ATP. T4 DNA ligase is a ligase that can ligate DNA-DNA, DNA-RNA, RNA-RNA and the sticky or blunt ends of double-stranded DNA. The molecular weight thereof is about 62 kD, and the activity thereof is easily inhibited by 0.2 mol/L KCl and spermine.

The term "restriction-ligation" refers to an assembly method in which multiple fragments can be ligated to each other in a specified order in one reaction. The complementary sticky ends are constructed on the fragments to be assembled, and then the fragments to be assembled are mixed with a restriction endonuclease (especially a type IIS restriction endonuclease) and a DNA ligase, and cycled between the high temperature (the optimal temperature for the restriction endonuclease, e.g., 37°C) and the low temperature (the optimal temperature for the ligase, e.g., 16°C) multiple times. High temperature favors enzymatic digestion and low temperature favors ligation. Each cycle can result in re-digestion of fragments that are not ligated or ligated back to the original plasmid, thereby retaining correctly assembled plasmids in a greater probability. Both enzymes are finally inactivated. The assembly method of restriction-ligation devised firstly by Golden Gate using type IIS restriction endonuclease *Bsa*I and a ligase is called Golden Gate cloning. For specific operations, see, for example, Engler C et al., PLoS ONE, 2008, 3(11): e3647 and Engler C et al., PLoS ONE, 2009, 4(5): e5553.

The term "introducing" refers to the insertion of an exogenous polynucleotide sequence into a DNA strand. The inserted exogenous polynucleotide sequence can usually be expressed normally.

The term "expression" refers to the process by which information (such as genetically encoded and/or epigenetic information) is transformed into a structure that exists and operates in a cell. Therefore, as used herein, "expression" can refer to transcription into a polynucleotide, translation into a polypeptide, or even polynucleotide and/or polypeptide modification (e.g., post-translational modification of a polypeptide). The fragment of transcribed polynucleotide, the fragment of translated polypeptide, or the fragment of polynucleotide and/or polypeptide modifications (e.g., post-translational modification of a polypeptide) should also be regarded as being expressed whether they are derived from a transcript generated by alternative splicing or a degraded transcript, or from a post-translational processing of the polypeptide (e.g., by proteolysis). An "expressed gene" includes genes that are transcribed into a polynucleotide (such as mRNA) and then translated into a polypeptide, as well as genes that are transcribed into RNA but not translated into a polypeptide (e.g., transfer and ribosomal RNA).

The term "PCR amplification" refers to the polymerase chain reaction, which is a molecular biology technique for amplifying a specific DNA fragment. It can be regarded as a special DNA replication *in vitro.* The biggest feature of PCR is that it can greatly increase the trace amount of DNA. Specifically, the basic principle of PCR technology is similar to the natural replication process of DNA, and its specificity relies on oligonucleotide primers complementary to both ends of the sequence to be amplified. The term "PCR template" refers to a specific DNA fragment to be amplified. The term "PCR primer (pair)" refers to a pair of oligonucleotide sequences, wherein the forward primer (F) is an oligonucleotide sequence complementary to the 5' end bases of the sense strand in double-stranded DNA as a template (A is complementary to T, C is complementary to G, and vice versa) and the reverse primer (R) is an oligonucleotide sequence complementary to the 3' end bases of the antisense strand in the double-stranded DNA as a template (A is complementary to T, C is complementary to G, and vice versa). PCR amplification is composed of three basic reaction steps: denaturation, annealing and extension, and comprises: (1) denaturation of a template DNA: heating the template DNA to above 90°C for a certain period of time, and then dissociating the double-stranded template DNA or the double-stranded DNA formed by PCR amplification to become single strands, so that the single strands can be bound with primers to prepare for the next round of reaction; (2) annealing (renaturation) of the template DNA and primers: after the template DNA is denatured into a single strand by heating, lowering the temperature to about 50°C, wherein the primers are paired and bound with the complementary sequence of the single-stranded template DNA; (3) extension of primers: synthesizing a new semiconservation replication strand complementary to the template DNA strand from the DNA template-primer conjugate, under the action of DNA polymerase at about 70°C, using dNTPs as the reaction raw material on the basis of the template, and according to the principles of complementary base pairing and semiconservation replication. More "semiconservation replication strands" can be obtained by repeating the cycle of three processes denaturationannealing-extension, and this new strand can become the template for the next cycle. The term "DNA polymerase" refers to a class of enzymes that use parental DNA as a template and catalyze the polymerization of substrate dNTP molecules to form daughter DNA. Common features of DNA polymerases are that: (1) the DNA polymerases have 5'→3' polymerase activity, which determines that DNA can only be synthesized along the 5'→3' direction; and (2) DNA polymerases cannot catalyze the *de novo* synthesis of new DNA strand, and can only catalyze the addition of dNTPs to the terminal 3'-OH of the nucleotide strand, therefore DNA primers are needed as the starting point for synthesis. High-fidelity DNA polymerase refers to a DNA polymerase with higher fidelity, and DNA synthesized thereby is more identical to the template than that synthesized by other DNA polymerases. Any high-fidelity DNA polymerase known in the art can be used in the present invention, such as Phusion DNA polymerase, Q5 polymerase, primerSTAR polymerase, *etc.*

When various molecular biological operations are carrying out for constructing the gene mutation library of the present invention, the procedures and methods (including but not limited to synthesis, enzyme digestion, ligation, PCR, recovery, purification, *etc*.) used are all well known to a person skilled in the art (see, e.g., Sambrook et al., 1989, Molecular Cloning: A Laboratory Manual (Second Edition), Cold Spring Harbor, New York).

The present invention provides a method for constructing a large-storage capacity gene mutation library, which solves the problem of extremely high cost for construction of a large-capacity gene mutation library in the prior art. The storage capacity of the gene mutation library constructed by the present invention can be up to 10⁵, preferably up to 10⁶, more preferably up to 10⁷, more preferably up to 10⁸, more preferably up to 10⁹, and more preferably up to 10¹⁰. In one specific embodiment, the storage capacity of the gene mutation library is 1.25 × 10⁷.

The amino acid sequence which requires library construction is provided and the positions and types of the amino acids which require to be mutated therein are determined. The storage capacity is obtained by calculation, and the nucleotide sequence encoding the amino acid sequence is determined. The sequence and position of the sticky ends in the coding nucleotide sequence is determined, and the coding nucleotide sequence is divided into two or more (such as, 3-10, preferably 3-6) fragments corresponding to the two or more (such as, 3-10, preferably 3-6) oligomer pools according to the sticky ends. Sicky ends are divided according to the number of sequences thereof into a single sticky end (which have only one sequence) and a degenerate sticky end that can divide a sequence between consecutive mutation points into two fragments (the degenerate sticky end has many different sequences, and the number of specific sequences depends on the mutation at the sticky end). The single sticky end can be suitable for dividing the coding nucleotide sequence into more than two fragments, such as two, three, four, five, six, seven, eight, nine and ten fragments. Sequence selection for the sticky end is on the basis of the following criteria: 1) the sticky end is in the middle of the coding nucleotide sequence, and thus the sequences in the oligomer pool can be synthesized as two fragments of approximately same length respectively; 2) there is no palindromic sequence in the sequence of the sticky end; 3) the GC content of the sticky end sequence is 50%-75%; and 4) the length of the sticky end matches the restriction site of the selected type IIS restriction endonuclease. The selection of sticky ends can also refer to literature, such as Vladimir P, Ong J L, Kucera R B et al., Optimization of Golden Gate assembly through application of ligation sequence-dependent fidelity and bias profiling[J]. BioRxiv, 2018: 322297. Each oligomer pool is assembled with appropriate sticky end/sticky end with low-mismatch rate, and then amplified with a forward primer of a first oligomer pool and a reverse primer of a last oligomer pool to obtain a final fragment.

The restriction site of the restriction endonuclease contained in the synthesized oligomer pool of the present invention is the restriction site of the IIS type restriction endonuclease, which is usually 1-6 bp, preferably 1-4 bp away from the recognition sequence. The type IIS restriction endonuclease is selected from *Acu*I*, Alw*I*, Bae*I*, Bbs*I*, Bbv*I*, Bcc*I*, Bce*AI*, Bcg*I*, Bci*VI*, Bfu*AI*, Bmr*I*, Bpm*I*, Bpu*EI*, Bsa*I*, Bsa*XI*, Bse*RI*, Bsg*I*, Bsm*AI*, BsmBI, Bsm*FI*, Bsp*MI*, Bsp*QI*, Bsr*DI*, Btg*ZI*, Bts*CI*, Bts*I*, Ear*I*, Eci*I*, Eco*P15I, *Fau*I*, Fok*I*, Hae*III*, Hga*I*, Hph*I*, HpyAV, Mbo*II*, Mly*I*, Mme*I*, Mnl*I*, Nme*AIII*, Ple*I*, Sap*I*,* and *Sfa*NI*.* Preferably, the type IIS restriction endonuclease is *Bsa*I and the recognition sequence thereof is GGTCTC.

The method of the present invention is further described in detail below with two fragments as an example:
after the coding nucleotide sequence is divided into two fragments, the reverse complement sequence of the recognition sequence of the IIS type restriction endonuclease (such as *Bsa*I) is added at the 3' end of the first DNA fragment, so that the sticky end determined above can be retained after enzyme digestion, then a specific sequence of 15-30 bp, preferably 15-25 bp, more preferably 18-21 bp is added at the 3' end of the DNA sequence with the recognition sequence introduced, so as to facilitate subsequent amplification; and the recognition sequence of the IIS type restriction endonuclease (such as *Bsa*I) and the sticky end sequence determined above are sequentially added at the 5' end of the second DNA fragment, so that the same sticky end as that of the first DNA fragment can be retained after enzyme digestion, and a specific sequence of 15-30 bp, preferably 15-25 bp, more preferably 18-21 bp is added at the 5' end of the DNA sequence with the recognition sequence and the sticky end introduced, so as to facilitate subsequent amplification. The specific sequence can be a random sequence that are not homologous to the original coding nucleotide sequence.

The first and second oligomer sequences can be designed and synthesized using any method known in the art, for example, using Library generate procedure. The number of the first and second sequences can be calculated according to the ratio and the total number of synthesized primers. Since the amount of synthesized oligomers is too small to meet the needs of subsequent operations, it is necessary to amplify the first and second oligomers. The amplification primers for oligomer pool-1 and oligomer pool-2 are designed by methods known in the art. The forward primer for oligomer pool-1 is named IF and the reverse primer is named 1R. The forward primer for oligomer pool-2 is named 2F and the reverse primer is named 2R. The length of the primers can be 15-30 bp, preferably 15-25 bp, more preferably 18-21 bp. The synthesized oligomer pool is amplified by using the synthesized oligomer pool-1 as a template and using 1F/1R as primers, or using oligomer pool-2 as a template and using 2F/2R as primers, respectively, and adding high-fidelity DNA polymerase (such as Phusion DNA polymerase), dNTPs, PCR buffer and optional bovine serum albumin (BSA). The amplified oligomer pool-1 is obtained with primers 1F/1R, and the amplified oligomer pool-2 is obtained with primers 2F/2R. After the amplification is completed, gel electrophoresis is used to detect whether the band is single. If the band is single, the amplified product is purified by column; or if the amplified band is not single, the PCR conditions need to be changed for re-amplification. The PCR conditions that need to be changed include: ratio of template to primer, annealing temperature, annealing time, the number of cycles, *etc.*

After the purified amplification product is obtained, the two oligomers are assembled by using the restriction-ligation method, and the restriction-ligation method is preferably performed by using the Golden Gate cloning. The preparation of an assembly system comprises: adding the purified first oligomer and second oligomer to the system, and simultaneously adding a restriction endonuclease and a DNA ligase (and/or the buffer thereof). The procedure of the assembly reaction is cycling two steps (37°C for 3 minutes and 16°C for 5 minutes) for 20 times. 37°C is the optimal reaction temperature for the restriction endonuclease, and 16°C is the optimal reaction temperature for the DNA ligase. In the assembly process, the mixture is reacted at 37°C for 3 minutes, wherein the restriction endonuclease recognizes and cuts the restriction site to produce the above-determined sticky end, then the mixture was reacted at 16°C for 5 minutes, wherein the DNA ligase ligates the two oligomers with same sticky end sequence. The correctly ligated fragment does not contain the recognition sequences of the restriction endonuclease and the incorrectly ligated fragment contains the recognition sequence of the restriction endonuclease. At 37°C of the next cycle, the restriction endonuclease can cut the incorrectly ligated fragment again until the fragment is correctly ligated. The mixture was finally reacted at 80°C for 15 minutes to inactivate the two enzymes, so as not to affect the subsequent reactions. The amount of DNA in the assembly system is very low, DNA can hardly be obtained after purification, and the system may contain unassembled fragments. Therefore, PCR amplification of the assembled sequence is required.

The assembled sequence pool is amplified by using the assembled unpurified product as a template, a forward primer IF of the first oligomer and a reverse primer 2R of the second oligomer and a high-fidelity DNA polymerase (such as Phusion DNA polymerase, *etc.*) to perform PCR, to obtain a large-storage capacity gene mutation library. The PCR product can then be recovered and/or purified to obtain a final product. The recovery and/or purification is preferably by gel electrophoresis. The final product is ligated via blunt ends and is ligated to a linearized plasmid (such as Puc57-EV), and Sanger sequencing is performed on the final product using universal primer (such as M13F primer) to check whether the sequence is correct; At the same time, the final product is subjected to high-throughput sequencing technology (NGS) to detect sequence distribution, amino acid distribution, *etc.*

### Description Of Sequences

| SEQ ID NO | Specific amino acids or sequences |
|---|---|
| 1 | RPDVNASX1X2X3GX4TPLHLAAX5X6GHLEIVEVLLKX7GADVRPD |
| 2 | AGACCTGACGTTAACGCTAGCX1X2X3GGTX4ACTCCGCTGCACCTGGCTGCTX5X6GGTCACCTGG AAATCGTTGAAGTTCTGCTGAAGX7GGTGCTGACGTGAGACCTGAC |
| 3 | AGACCTGACGTTAACGCTAGCX1X2X3GGTX4ACTCCGCTGCACCTG |
| 4 | GAGACC |
| 5 | AGACCTGACGTTAACGCTAGCX1X2X3GGTX4ACTCCGCTGCACCTGAGAGACC |
| 6 | *ACCTACGTCCAAATCGGCTG* |
| 7 | AGACCTGACGTTAACGCTAGCX1X2X3GGTX4ACTCCGCTGCACCTGAGAGACCACCTACGTCCAAA TCGGCTG |
| 8 | GCTGCTX5X6GGTCACCTGGAAATCGTTGAAGTTCTGCTGAAGX7GGTGCTGACGTGAGACCTGAC |
| 9 | GGTCTC |
| 10 | GGTCTCGCCTGGCTGCTX5X6GGTCACCTGGAAATCGTTGAAGTTCTGCTGAAGX7GGTGCTGACGT GAGACCTGAC |
| 11 | *ACTCCTAAGTAAAATGTGCAC* |
| 12 | ACTCCTAAGTAAAATGTGCACGGTCTCGCCTGGCTGCTX5X6GGTCACCTGGAAATCGTTGAAGTTC TGCTGAAGX7GGTGCTGACGTGAGACCTGAC |
| 13 | 1F: AGACCTGACGTTAACGCTAG |
| 14 | 1R: CAGCCGATTTGGACGTAGGT |
| 15 | 2F: ACTCCTAAGTAAAATGTGCA |
| 16 | 2R: GTCAGGTCTCACGTCAGCAC |
| 17 | AGACCTGACGTTAACGCTAGCX1X2X3GGTX4ACTCCGCTGCACCTGGCTGCTX5X6GGTCACCTGG AAATCGTTGAAGTTCTGCTGAAGX7GGTGCTGACGTGAGACCTGAC |
| 18 | KTEDTAVYYCSRD102103104105106107DAWGQGTLVTVSS |
| 19 | AAAACCGAGGACACGGCCGTGTATTACTGTAGTAGAGAT102103104105106107GACGCCTGGG GCCAAGGAACCCTGGTCACCGTCTCCTCG |
| 20 | AAAACCGAGGACACGGCCGTGTATTACTGTAGTAGAGAT102103104105 |
| 21 | AAAACCGAGGACACGGCCGTGTATTACTGTAGTAGAGAT102103104105AGAGACC |
| 22 | AAAACCGAGGACACGGCCGTGTATTACTGTAGTAGAGAT102103104105AGAGACC ACCTACGTCCAAATCGGCTG |
| 23 | 104105106107GACGCCTGGGGCCAAGGAACCCTGGTCACCGTCTCCTCG |
| 24 | GGTCTCG104'105106107GACGCCTGGGGCCAAGGAACCCTGGTCACCGTCTCCTCG |
| 25 | ACTCCTAAGTAAAATGTGCACGGTCTCG104’105106107GACGCCTGGGGCCAAGGAACCCTGGT CACCGTCTCCTCG |
| 26 | 1sF: AAAACCGAGGACACGGCCGT |
| 14 | 1sR: CAGCCGATTTGGACGTAGGT |
| 15 | 2sF: ACTCCTAAGTAAAATGTGCA |
| 27 | 2sR: CGAGGAGACGGTGACCAGGG |

The technical solutions of the present invention are further illustrated in more detail by the examples and in conjunction with the accompanying drawings, however, the present invention is not limited to the following examples.

### Specific Examples

### Example 1. Design of an oligomer pool having a mutant nucleotide and a restriction site of a restriction endonuclease

### 1.1 Providing the amino acid sequence which required library construction and determining the positions and types of the amino acids which required to be mutated therein

The amino acid sequence which required library construction was as follows: RPDVNASX1X2X3GX4TPLHLAAX5X6GHLE IVEVLLKX7GADVRPD (SEQ ID NO: 1), wherein X1-X7 were amino acids which required to be mutated, and X1 was mutated to 4 of the 20 amino acids, X2-X6 were mutated to 16 of the 20 amino acids, and X7 was mutated to 3 of the 20 amino acids, and therefore, the storage capacity of the library was 4 × 16 × 16 × 16 × 16 × 16 × 3 = 12582912. The nucleotide sequence encoding the amino acid sequence was as follows: AGACCTGACGTTAACGCTAGCX1X2X3GGTX4ACTCCGCTGCA**CCTG**GCTG CTX5X6GGTCACCTGGAAATCGTTGAAGTTCTGCTGAAGX7GGTGCTGACG TGAGACCTGAC (SEQ ID NO: 2), wherein each of X1-X7 represented 1 codon (3 nucleotides) to be mutated.

### 1.2 Dividing the sequence into fragments

The method for dividing the sequence into fragments was described in detail by taking a single sticky end as an example.

The coding nucleotide sequence was divided into two fragments according to a single sticky end. The sequence of the sticky end was selected according to the position of the mutant nucleotide on the basis of the following criteria: 1) the sticky end was in the middle of the oligomer sequence, and can divide the sequence in the oligomer pool into two fragments of approximately same length for separate synthesis; 2) there was no palindromic sequence in the sequence of the sticky end; 3) the GC content of the sticky end sequence was 50%-75%; and 4) the length of the sticky end matched the restriction site of the selected type IIS restriction endonuclease.

For the coding nucleotide sequence of 1.1 and on the basis of the above criteria, 1) CCTG was located at positions 48-51 of 120 nucleotides; 2) there was no palindromic structure in CCTG; 3) the GC content of CCTG was 75%; and 4) CCTG had 4 nucleotides, and corresponded to the restriction site of type IIS restriction endonuclease *Bsa*I*,* therefore CCTG (shown in bold in the above sequence) was selected as the sticky end for dividing the sequence into two fragments.

### 1.3 Design of each oligomer pool via fragmentation

The first oligomer is 51 bp in length, and the specific sequence thereof was as follows: 5'-AGACCTGACGTTAACGCTAGCX1X2X3GGTX4ACTCCGCTGC **ACCTG-**3' (SEQ ID NO: 3). The reverse complement sequence GAGACC (SEQ ID NO: 4) of the recognition sequence GGTCTC of *Bsa*I was added at the 3' end of the first DNA sequence. According to the specific restriction site of *Bsa*I*,* base A was additionally added at the 5' end of the reverse complement sequence GAGACC of the recognition sequence of *Bsa*I in order to generate the selected sticky end CCTG after digestion with *Bsa*I*.* The sequence with the restriction site introduced was AGACCTGACGTTAACGCTAGCX1X2X3GGTX4ACTCCGCTGCA**CCTG*A***GAG ACC (SEQ ID NO: 5). A specific sequence of 18-21 bp was further added at the 3' end of the above sequence for subsequent amplification. The specific sequence was, for example *ACCTACGTCCAAATCGGCTG* (SEQ ID NO: 6). The final sequence of the first oligomer was AGACCTGACGTTAACGCTAGCX1X2X3GGTX4ACTCCGC TGCACCTGAG***A***GACC*ACCTACGTCCAAATCGGCTG* (SEQ ID NO: 7), which was named oligomer pool-1.

The length of the second oligomer was 69 bp, and the specific sequence thereof was as follows: 5'-GCTGCTX5X6GGTCACCTGGAAATCGTTGAAGTTCTGC TGAAGX7GGTGCTGACGTGAGACCTGAC-3' (SEQ ID NO: 8). Sticky end sequence CCTG and recognition sequence GGTCTC (SEQ ID NO: 9) of restriction endonuclease *Bsa*I were sequentially added at the 5' end of the second DNA sequence. According to the specific restriction site of *Bsa*I*,* base G was additionally added at the 3' end of the recognition sequence GGTCTC of *Bsa*I in order to generate the selected sticky end CCTG after digestion with *Bsa*I*.* The obtained sequence was GGTCTC***G*CCTG**GCTGCTX5X6GGTCACCTGGAAATCGTTGAAGTTCTGCTG AAGX7GGTGCTGACGTGAGACCTGAC (SEQ ID NO: 10). A specific sequence of 18-21 bp was further added at the 5' end of the above sequence for subsequent amplification. The specific sequence was, for example *ACTCCTAAGTAAAATGTGCAC* (SEQ ID NO: 11). The final sequence of the second oligomer was *ACTCCTAAGTAAAATGTGCAC*GGTCTC***G*CCTG**GCTGCTX5X6GGTCACCTGGA AATCGTTGAAGTTCTGCTGAAGX7GGTGCTGACGTGAGACCTGAC (SEQ ID NO: 12), which was named oligomer pool-2.

### Example 2. Design of oligomer pools by Library generate procedure

A single sticky end was taken as an example. An oligomer pool was generally designed by Library generate procedure.

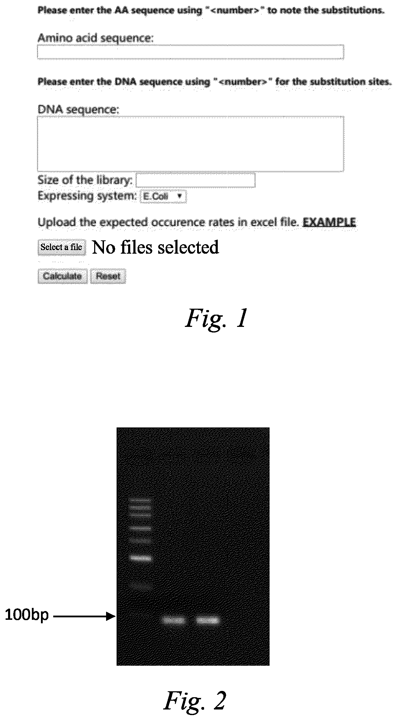

The website of library generate procedure is http://10.1.1.25/cgi-bin/aa_lib_generate.py, and the interface of Library generate procedure for designing an oligomer pool is shown in Fig. 1.

The procedure can design 1 oligomer pool per run, and oligomer pool-1 and oligomer pool-2 needed to be designed separately. An exemplary design process for oligomer pool-1 was as follows:
entering the amino acid sequence which required library construction in Amino acid sequence input box, wherein the mutation sites were respectively represented by numbers <1>, <2>, <3>, *etc.,* one number represents one amino acid, and for oligomer pool-1, the entered amino acid sequence was RPDVNAS<1><2><3>G<4>TPLHLRDHLRPNRL;
entering the DNA sequence encoding the amino acid sequence in DNA sequence input box, wherein the mutation sites were respectively represented by numbers <1>, <2>, <3>, *etc.,* with one number representing one codon, and for oligomer pool-1, the entered DNA sequence was AGACCTGACGTTAACGCTAGC<1><2><3>GGT <4>ACTCCGCTGCA CCTGAGAGACCACCTACGTCCAAATCGGCTG;
entering the number of sequences in the library in Size of the library input box, wherein for oligomer pool-1, <1> was mutated to 4 of the 20 amino acids, <2>-<4> were mutated to 16 of the 20 amino acids, the number of oligomers to be synthesized in oligomer pool-1 was 4 × 16 × 16 × 16 = 16384, therefore 16384 was entered;
selecting the desired expression system in Expressing system input box, for example, selecting *"E. coli"* if the amino acid sequence was expressed in *E. coli,* selecting "Human" if the amino acid sequence was expressed in human cells, *etc.,* wherein for oligomer pool-1, human codons were used and "Human" was selected;
and optionally uploading a pre-stored excel file in "Upload the expected occurrence rates in excel file" option, wherein the occurrence frequency of each mutated amino acid in oligomer pool-1 can be set in the file.

Similarly, an exemplary design process for oligomer pool-2 was as follows:
entering the amino acid sequence which required library construction in Amino acid sequence input box, wherein the mutation sites were respectively represented by numbers <1>, <2>, <3>, *etc.,* one number represents one amino acid, and for oligomer pool-2, the entered amino acid sequence was LLSKMCRSRLAA<1><2>GHLEIVEVLLK<3> GADVRPD;
entering the DNA sequence encoding the amino acid sequence in DNA sequence input box, wherein the mutation sites were respectively represented by numbers <1>, <2>, <3>, *etc.,* one number represents one codon, and for oligomer pool-2, the entered DNA sequence was CTCCTAAGTAAAATGTGCAGGTCTCGCCTGGCTGCT <1><2>GGTCACCTGGAAATCGTTGAAGTTCTGCTGAAG<3>GGTGCTGACG TGAGACCTGAC.
entering the number of sequences in the library in Size of the library input box, wherein for oligomer pool-2, <1>-<2> were mutated to 16 of the 20 amino acids, <3> was mutated to 3 of the 20 amino acids, the number of oligomers to be synthesized in oligomer pool-2 was 16 × 16 × 3 = 768, therefore 768 was entered;
selecting the desired expression system in Expressing system input box, wherein for oligomer pool-2, human codons were used and "Human" was selected;
and optionally uploading a pre-stored excel file in "Upload the expected occurrence rates in Excel file" option, wherein the occurrence frequency (expected value) of each mutated amino acid in oligomer pool-2 can be set in the file.

**Table 1. Setting of the occurrence frequency of each mutant amino acid in oligomer pool-1 and oligomer pool-2**

| | X1 | X2 | X3 | X4 | X5 | X6 | X7 |
|---|---|---|---|---|---|---|---|
| Alanine A | | 7% | 7% | 7% | 7% | 7% | |
| Cysteine C | | | | | | | |
| Aspartic acid D | 55% | 7% | 7% | 7% | 7% | 7% | |
| Glutamic acid E | | 7% | 7% | 7% | 7% | 7% | |
| Phenylalanine F | | 4% | 4% | 4% | 4% | 4% | |
| Glycine G | | | | | | | |
| Histidine H | | 7% | 7% | 7% | 7% | 7% | 33% |
| Isoleucine I | | 4% | 4% | 4% | 4% | 4% | |
| Lysine K | | 7% | 7% | 7% | 7% | 7% | |
| Leucine L | | 4% | 4% | 4% | 4% | 4% | |
| Methionine M | | | | | | | |
| Asparagine N | 15% | 7% | 7% | 7% | 7% | 7% | 33% |
| Proline P | | | | | | | |
| Glutamine Q | | 7% | 7% | 7% | 7% | 7% | |
| Arginine R | | 7% | 7% | 7% | 7% | 7% | |
| Serine S | 15% | 7% | 7% | 7% | 7% | 7% | |
| Threonine T | 15% | 7% | 7% | 7% | 7% | 7% | |
| Valine V | | 4% | 4% | 4% | 4% | 4% | |
| Tryptophan W | | 4% | 4% | 4% | 4% | 4% | |
| Tyrosine Y | | 10% | 10% | 10% | 10% | 10% | 33% |

### Example 3. Synthesis of oligomer pool

The oligomer pool 1 and the oligomer pool-2 designed in Example 1 or Example 2 were synthesized by various synthetic methods known in the art. The methods included, but are not limited to, phosphotriester method, phosphoramidite method, hydrogen phosphoric acid method, synthesis by chip, *etc.*

In the present invention, a semiconductor chip (B3P synthesizer from CustomArray) was used to synthesize oligomer pool 1 and oligomer pool 2. Synthesis of an oligomer pool only required synthesis of a chip with a capacity of 92 k (92,000 sequences) in the case of dividing into two fragments. In the case of not dividing into two fragments, multiple chips were required to synthesize an oligomer pool for a long sequence with a storage capacity of 1.26 × 10⁷. Chips were expensive. Therefore, the construction of an oligomer pool via fragmentation greatly reduced the construction cost.

### Example 4. Amplification of a synthesized oligomer pool using high-fidelity DNA polymerase

According to the nucleotide sequences of oligomer pool-1 and oligomer pool-2 obtained above, the amplification primers for oligomer pool-1 and oligomer pool-2 were designed by methods known in the art. The forward primer for oligomer pool-1 was named IF and the reverse primer was named 1R. The forward primer for oligomer pool-2 was named 2F and the reverse primer was named 2R. The sequence of each primer was as follows:
1F: AGACCTGACGTTAACGCTAG (SEQ ID NO: 13)
1R: CAGCCGATTTGGACGTAGGT (SEQ ID NO: 14)
2F: ACTCCTAAGTAAAATGTGCA (SEQ ID NO: 15)
2R: GTCAGGTCTCACGTCAGCAC (SEQ ID NO: 16)

The PCR reaction system was configured as follows: the synthesized oligomer pool was used as a template, 1F/1R or 2F/2R was used as primers, high-fidelity DNA polymerase (such as Phusion DNA polymerase), dNTPs, 5x HF buffer, and bovine serum albumin (BSA) were added, and water was used for making up the system to 50 µl; the synthesized oligomer pool was amplified and the amplified oligomer pool-1 was obtained with primers 1F/1R, and the amplified oligomer pool-2 was obtained with primers 2F/2R.

The reaction system was as follows:

| | |
|---|---|
| HF buffer (5x) | 10 µl |
| 10 nM dNTP | 1 µl |
| Template | 100 ng |
| Forward/reverse primer | 2 µl/2 µl |
| BSA (20 mg/ml) | 10 µl |
| Phusion (2 U/µl) | 0.5 µl |
| H₂O | Added to 50 µl |

The reaction procedure was as follows:

| | |
|---|---|
| 1 | 98°C 30 seconds |
| 2 | 98°C 10 seconds |
| 3 | 64°C 10 seconds |
| 4 | 72 °C 20 seconds |
| 5 | return to step 2, 16 cycles |
| 6 | 72°C 5 minutes |

### Example 5. Assembly of two oligomer pools using Golden Gate

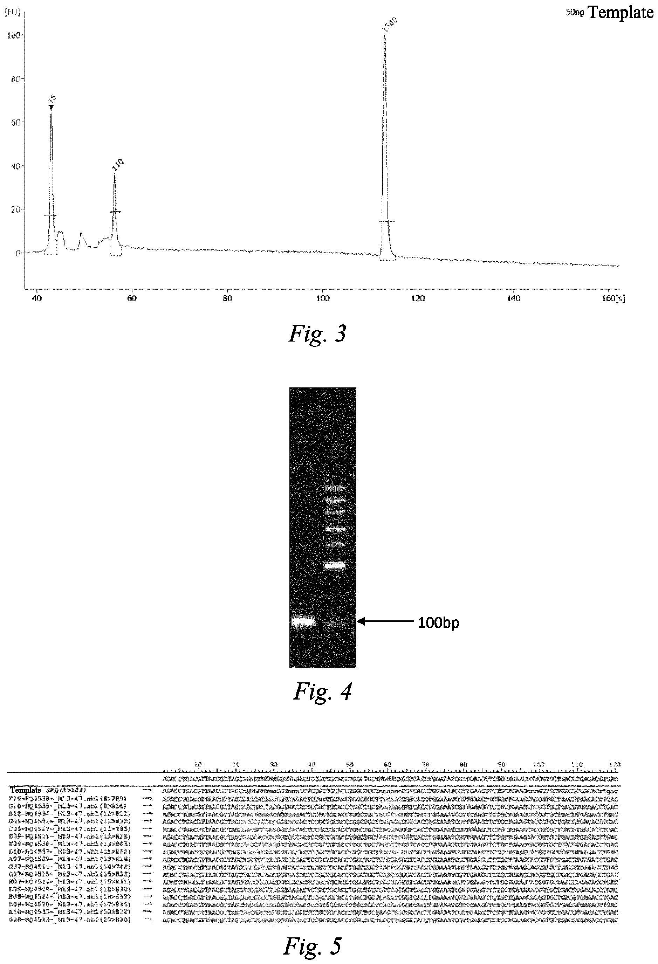

The preparation of a Golden Gate assembly system comprises: adding oligomer pool-1 and oligomer pool-2, simultaneously adding IIS type restriction endonuclease *Bsa*I and T4 DNA ligase and 10x T4 DNA ligase buffer to the reaction system, making up the reaction system to 20 µl with water, and assembling two oligomer pools. The assembled product was detected by Agilent 2100, and the detection result was shown in Fig. 3.

The reaction system was as follows:

| | |
|---|---|
| Oligomer pool-1 | 50 ng |
| Oligomer pool-2 | 50 ng |
| T4 DNA ligase buffer | 2 µl |
| T4 DNA ligase | 1 µl |
| *Bsa*I | 1 µl |
| H₂O | Added to 20 µl |

37°C was the optimal reaction temperature for the restriction endonuclease *Bsa*I*,* and 16°C was the optimal reaction temperature for the T4 DNA ligase. In the reaction procedure, the mixture was reacted at 37°C for 3 minutes, wherein the restriction endonuclease *Bsa*I recognized and cut the *Bsa*I restriction sites in the sequences of oligomer pool-1 and oligomer pool-2 and a 4 bp sticky end was left in each sequence, and then the mixture was reacted at 16°C for 5 minutes, wherein T4 DNA ligase ligated the two oligomer pools at sticky ends. The correctly ligated fragments (*i.e.,* fragments formed by the ligation of oligomer pool-1 with oligomer pool-2) did not contain *Bsa*I recognition sequence, and the incorrectly ligated fragments (i.e., fragments formed by the ligation of oligomer pool-1 with oligomer pool-1 or oligomer pool-2 with oligomer pool-2) contained *Bsa*I recognition sequence. When the mixture was reacted at 37°C again, restriction endonuclease *Bsa*I can cut the incorrectly ligated fragments again until the fragments were connected correctly. After 20 cycles, the mixture was reacted at 80°C for 15 minutes to inactivate *Bsa*I and T4 DNA ligase, so as not to affect the subsequent reactions. The assembled oligomer pool was finally obtained.

The reaction procedure was as follows:

| | |
|---|---|
| 1 | 37°C 3 minutes |
| 2 | 16°C 5 minutes |
| 3 | return to step 1, 20 cycles |
| 4 | 80°C 15 minutes |
| 5 | 4°C ∞ |

### Example 6. Amplification of the assembled oligomer pool

The sequence of the assembled oligomer pool was shown in SEQ ID NO: 17. However, the amount of DNA in the system after assembly was very low, DNA can hardly be obtained after purification, and the system may contain unassembled fragments. Therefore, PCR amplification of the assembled sequence was required. The PCR reaction system was configured as follows: the assembled oligomer pool was used as a template, 1F/2R were used as forward and reverse primers, high-fidelity DNA polymerase (such as Phusion DNA polymerase), dNTPs, and 5x HF buffer were added, water was used for making up the system to 50 µl and the assembled oligomer pool was amplified.

The reaction system was as follows:

| | |
|---|---|
| HF buffer (5x) | 10 µl |
| 10 nM dNTP | 1 µl |
| Template (assembled product) | 2.5 ul |
| Primer | 1 µl/1 µl |
| Phusion (2 U/µl) | 0.5 µl |
| H₂O | Added to 50 µl |

The reaction procedure was as follows:

| | |
|---|---|
| 1 | 98°C 30 seconds |
| 2 | 98°C 10 seconds |
| 3 | 60°C 30 seconds |
| 4 | 72°C 20 seconds |
| 5 | return to step 2, 25 cycles |
| 6 | 72°C 5 minutes |

Finally, the gene mutation library product was obtained.

### Example 7. Purification and sequencing of gene mutation library product

The product obtained in Example 6 was recovered and purified by agarose gel electrophoresis to obtain the final library product. The electropherogram of the final library product is shown in Figure 4.

The blunt end of the final library product obtained by assembling the single sticky end were ligated to the linearized plasmid Puc57-EV, and Sanger sequencing was performed on the final product using universal primer M13F to check whether the sequence was correct; and high-throughput sequencing (NGS) was performed to detect sequence distribution, amino acid distribution, *etc.* The sequencing results are shown in Fig. 5-Fig. 7 and Table 1 below.

Fig. 5 is a Sanger sequencing result of the library sequence obtained by assembling a single sticky end, which proves that the desired gene mutation library is obtained.

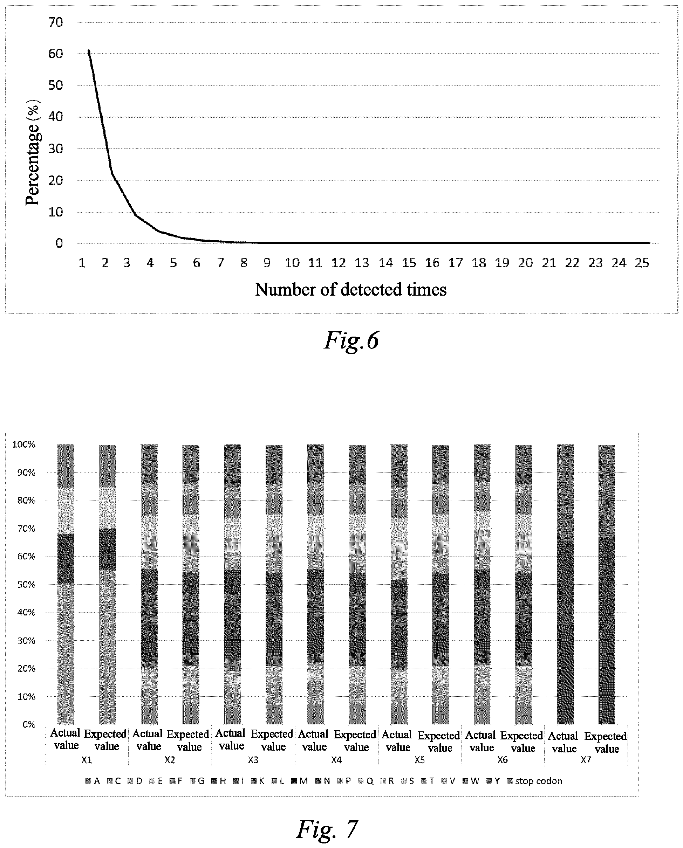

Fig. 6 is a sequence distribution diagram of NGS sequencing of the library sequence obtained by assembling a single sticky end, and Table 1 is a sequence distribution table of NGS sequencing of the library sequence obtained by assembling a single sticky end.

**Table 2. Sequence distribution, by NGS sequencing, of the library sequence obtained by assembling a single sticky end**

| Number of detected times of sequences | % of total number of sequences | Count |
|---|---|---|
| 1 | 60.962325 | 4331739 |
| 2 | 22.300805 | 1584606 |
| 3 | 9.059291 | 643717 |
| 4 | 3.974710 | 282427 |
| 5 | 1.852300 | 131617 |
| 6 | 0.894773 | 63579 |
| 7 | 0.448182 | 31846 |
| 8 | 0.229959 | 16340 |
| 9 | 0.122565 | 8709 |
| 10 | 0.066891 | 4753 |
| 11 | 0.037506 | 2665 |
| 12 | 0.020927 | 1487 |
| 13 | 0.012328 | 876 |
| 14 | 0.007206 | 512 |
| 15 | 0.004447 | 316 |
| 16 | 0.002505 | 178 |
| 17 | 0.001393 | 99 |
| 18 | 0.000802 | 57 |
| 19 | 0.000394 | 28 |
| 20 | 0.000310 | 22 |
| 21 | 0.000169 | 12 |
| 22 | 0.000113 | 8 |
| 23 | 0.000056 | 4 |
| 24 | 0.000028 | 2 |
| 25 | 0.000014 | 1 |

Fig. 7 is an amino acid distribution of NGS sequencing of the library sequence obtained by assembling a single sticky end. It can be seen from the above data, the distribution of the location, type and frequency of amino acid mutations in the library sequence is fully consistent with the expected design.

### Example 8. Obtaining gene mutation library by using a degenerate sticky end for assembly

### 8.1 Providing the amino acid sequence which required library construction and determining the positions and types of the amino acids which required to be mutated therein

The amino acid sequence which required library construction was as follows: KTEDTAVYYCSRD102103104105106107 DAWGQGTLVTVSS (SEQ ID NO: 18), wherein AA102-AA107 were amino acids which required to be mutated, AA102-AA106 was mutated to 18 of the 20 amino acids, and AA107 was mutated to 4 of the 20 amino acids, and therefore, the storage capacity of the library was 18 × 18 × 18 × 18 × 18 × 4 = 7558272. The nucleotide sequence encoding the amino acid sequence was as follows: AAAACCGAGGACACGGCCGTGTATTACTGTAGTAGAGAT 102103104105106107GACGCCTGGGGCCAAGGAACCCTGGTCACCGTCTCCT CG (SEQ ID NO: 19), wherein each of AA102-AA107 represented 1 codon (3 nucleotides) to be mutated.

### 8.2 Design of each oligomer pool via fragmentation

The coding nucleotide sequence was divided into two fragments, and the degenerate sticky end was chosen as the last base of AA104 and the three bases of AA105.

The first oligomer is 51 bp in length, and the specific sequence thereof was as follows: 5'-AAAACCGAGGACACGGCCGTGTATTACTGTAGTAGAGAT 102103104105-3' (SEQ ID NO: 20). The reverse complement sequence GAGACC (SEQ ID NO: 4) of the recognition sequence GGTCTC of *Bsa*I was added at the 3' end of the first DNA sequence. In order to cut accurately and avoid the introduction of unnecessary sequence, a base A was additionally added at the 5' end of the reverse complement sequence GAGACC of the recognition sequence of *Bsa*I*.* The sequence with the restriction site introduced was AAAACCGAGGACACG GCCGTGTATTACTGTAGTAGAGAT102103104105AGAGACC (SEQ ID NO: 21). A specific sequence of 18-21 bp was further added at the 3' end of the above sequence for subsequent amplification. The specific sequence was, for example *ACCTACGTCCAAATCGGCTG* (SEQ ID NO: 6). The final sequence of the first oligomer was AAAACCGAGGACACGGCCGTGTATTACTGTAGTAGAGAT 102103104105***A*** GAGACC *ACCTACGTCCAAATCGGCTG* (SEQ ID NO: 22), which was named oligomer pool-1.

Sequence design by Library generate required input of amino acid sequence and DNA sequence, the first two bases of AA104 needed to be added when the second oligomer was designed, therefore the second oligomer was 51 bp in length, and the specific sequence thereof was as follows: 5'-104105106107GACGCCTGGGGCCAAGGAACCCTGGTC ACCGTCTCCTCG-3' (SEQ ID NO: 23). After the sequence was designed, the first two bases of AA104 were removed. All sequences should be designed by using Library generate, and then DNA sequences such as *Bsa*I restriction site/specific sequence should be added due to the particularity of the second oligonucleotide (the first two bases of AA104 needed to be removed).

### 8.3. Design of oligomer pools by Library generate procedure

The website of library generate procedure is http://10.1.1.25/cgi-bin/aa_lib_generate.py, and the interface of Library generate procedure for designing an oligomer pool is shown in Fig. 1.

The procedure can design 1 oligomer pool per run, and oligomer pool-1 and oligomer pool-2 needed to be designed separately. An exemplary design process for oligomer pool-1 was as follows:
entering the amino acid sequence which required library construction in Amino acid sequence input box, wherein the mutation sites were respectively represented by numbers <1>, <2>, <3>, *etc.,* one number represents one amino acid, and for oligomer pool -1, the entered amino acid sequence was KTEDTAVYYCSRD<1><2><3><4>RDH LRPNRL;
entering the DNA sequence encoding the amino acid sequence in DNA sequence input box, wherein the mutation sites were respectively represented by numbers <1>, <2>, <3>, *etc.,* one number represents one codon, and for oligomer pool-1, the entered DNA sequence was AAAACCGAGGACACGGCCGTGTATTACTGTAGT AGAGAT<1><2><3><4> AGAGACCACCTACGTCCAAATCGGCTG;
entering the number of sequences in the library in Size of the library input box, wherein for oligomer pool-1, <1>-<4> were mutated to 18 of the 20 amino acids, the number of oligomers to be synthesized in oligomer pool-1 was 18 × 18 × 18 × 18 = 104976, therefore 104976 was entered;
selecting the desired expression system in Expressing system input box, for example, selecting *"E. coli"* if the amino acid sequence was expressed in *E. coli,* selecting "Human" if the amino acid sequence was expressed in human cells, *etc.,* wherein for oligomer pool-1, human codons were used and "Human" was selected;
and optionally uploading a pre-stored excel file in "Upload the expected occurrence rates in excel file" option, wherein the occurrence frequency of each mutated amino acid in oligomer pool-1 can be set in the file.

The sticky end of sequence 1 after digestion with *Bsa*I was located at the last base of AA104 and the three bases of AA105, and was a degenerate sticky end, specifically CAAC/CAAG/CACC/CAGC/CATC/CCAC/CCAG/CCCC/CCGG/CCTG/CGAC/CG AG/CGCC/CGGC/CGTG/CTAC/CTGG/CTTC/GAAC/GAAG/GACC/GAGC/GATC /GCAC/GCAG/GCCC/GCGG/GCTG/GGAC/GGAG/GGCC/GGGC/GGTG/GTAC/G TGG/GTTC, a total of 36 kinds.

Similarly, an exemplary design process for oligomer pool-2 was as follows:
entering the amino acid sequence which required library construction in Amino acid sequence input box, wherein the mutation sites were respectively represented by numbers <1>, <2>, <3>, *etc.,* one number represents one amino acid, and for oligomer pool-2, the entered amino acid sequence was <1><2><3><4>DAWGQGTLVTVSS;
entering the DNA sequence encoding the amino acid sequence in DNA sequence input box, wherein the mutation sites were respectively represented by numbers <1>, <2>, <3>, *etc.,* one number represents one codon, and for oligomer pool-2, the entered DNA sequence was <1><2><3><4>GACGCCTGGGGCCAAGGAACC CTGGTCACCGTCTCCTCG;
entering the number of sequences in the library in Size of the library input box, wherein for oligomer pool-2, <1>-<3> were mutated to 18 of the 20 amino acids, <4> was mutated to 4 of the 20 amino acids, the number of oligomers to be synthesized in oligomer pool-2 was 18 × 18 × 18 × 4 = 23328, therefore 23328 was entered;
selecting the desired expression system in Expressing system input box, wherein for oligomer pool-2, human codons were used and "Human" was selected;
and optionally uploading a pre-stored excel file in "Upload the expected occurrence rates in excel file" option, wherein the occurrence frequency (expected value) of each mutated amino acid in oligomer pool-2 can be set in the file.

After the software output the sequence, excel was used to process the obtained data. The column function in excel was used to separate the first two bases of AA104 and the sequence thereafter. AA104 with the first two bases were deleted was recorded as 104'. Then, sticky end sequence CCTG and recognition sequence GGTCTC (SEQ ID NO: 9) of restriction endonuclease *Bsa*I were sequentially introduced at the 5' end of the second DNA sequence. In order to cut accurately and avoid the introduction of unnecessary sequence, a base G was additionally added at the 3' end of the recognition sequence GGTCTC of *Bsa*I*.* The obtained sequence was GGTCTC***G***104'105106107GACGCCTGGGGCCAAGGAACCCTGGTCACCGTCT CCTCG (SEQ ID NO: 24). A specific sequence of 18-21 bp was further added at the 5' end of the above sequence for subsequent amplification. The specific sequence was, for example *ACTCCTAAGTAAAATGTGCAC* (SEQ ID NO: 11). The final sequence of the second oligomer was *ACTCCTAAGTAAAATGTGCAC*GGT CTC***G***104'105106107GACGCCTGGGGCCAAGGAACCCTGGTCACCGTCTCCT CG (SEQ ID NO: 25), which was named oligomer pool-2. The sticky end generated by oligomer pool-2 after digestion with *Bsa*I was the same as that generated by oligomer pool-1, and specifically was CAAC/CAAG/CACC/CAGC/CATC/CCAC/CCAG/CCCC/CCGG/CCTG/CGAC/CG AG/CGCC/CGGC/CGTG/CTAC/CTGG/CTTC/GAAC/GAAG/GACC/GAGC/GATC /GCAC/GCAG/GCCC/GCGG/GCTG/GGAC/GGAG/GGCC/GGGC/GGTG/GTAC/G TGG/GTTC, a total of 36 kinds. Oligomer pool-1 and oligomer pool-2 were ligated via same sticky end.

**Table 3. Setting of the occurrence frequency of each mutant amino acid in oligomer pool-1 and oligomer pool-2**

| | AA102 | AA103 | AA104 | AA105 | AA106 | AA107 |
|---|---|---|---|---|---|---|
| Alanine A | 7% | 7% | 7% | 7% | 7% | |
| Cysteine C | | | | | | |
| Aspartic acid D | 6% | 6% | 6% | 6% | 6% | |
| Glutamic acid E | 3% | 3% | 3% | 3% | 3% | |
| Phenylalanine F | 3% | 3% | 3% | 3% | 3% | 25% |
| Glycine G | 17% | 17% | 17% | 17% | 17% | |
| Histidine H | 2% | 2% | 2% | 2% | 2% | |
| Isoleucine I | 3% | 3% | 3% | 3% | 3% | 25% |
| Lysine K | 1% | 1% | 1% | 1% | 1% | |
| Leucine L | 5% | 5% | 5% | 5% | 5% | 25% |
| Methionine M | | | | | | 25% |
| Asparagine N | 3% | 3% | 3% | 3% | 3% | |
| Proline P | 5% | 5% | 5% | 5% | 5% | |
| Glutamine Q | 2% | 2% | 2% | 2% | 2% | |
| Arginine R | 7% | 7% | 7% | 7% | 7% | |
| Serine S | 11% | 11% | 11% | 11% | 11% | |
| Threonine T | 6% | 6% | 6% | 6% | 6% | |
| Valine V | 5% | 5% | 5% | 5% | 5% | |
| Tryptophan W | 4% | 4% | 4% | 4% | 4% | |
| Tyrosine Y | 10% | 10% | 10% | 10% | 10% | |

### 8.4 Construction of the gene library with a degenerate sticky end

The oligomer pools were synthesized according to the method of Example 3 of the present invention. According to the nucleotide sequences of oligomer pool-1 and oligomer pool-2 obtained above, the amplification primers for oligomer pool-1 and oligomer pool-2 were designed by methods known in the art. The forward and reverse primers of oligomer pool-1 were named IsF and IsR, respectively. The forward and reverse primers of oligomer pool-2 were named 2sF and 2sR, respectively. The sequence of each primer was as follows:
IsF: AAAACCGAGGACACGGCCGT (SEQ ID NO: 26)
1sR: CAGCCGATTTGGACGTAGGT (SEQ ID NO: 14)
2sF: ACTCCTAAGTAAAATGTGCA (SEQ ID NO: 15)
2sR: CGAGGAGACGGTGACCAGGG (SEQ ID NO: 27)

The synthesized oligomer pool was amplified by using the synthesized oligomer pool as a template, using 1sF/1sR or 2sF/2sR as primers, adding high-fidelity DNA polymerase (such as Phusion DNA polymerase) and according to the method of Example 4 of the present invention. The amplified oligomer pool-1 was obtained with primers 1sF/1sR, and the amplified oligomer pool-2 was obtained with primers 2sF/2sR.

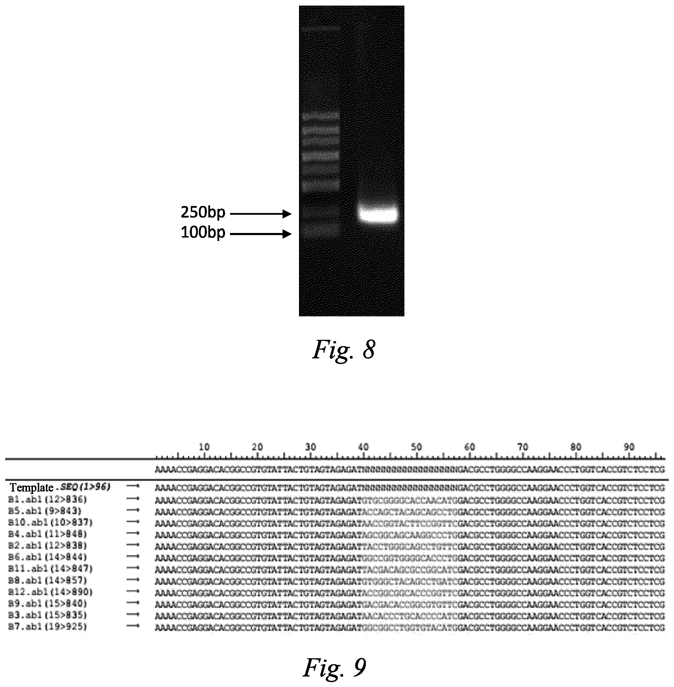

The two oligomer pools were assembled using Golden Gates method according to the method of Example 5 of the present invention. The assembled oligomer pool was amplified according to the method of Example 6 of the present invention, and the electrophoretogram of the final library product is shown in Fig. 8. The gene library product was purified and sequenced according to the method of Example 7 of the present invention. The sequencing results are shown in Fig. 9-Fig. 11 and Table 2 below.

Fig. 9 is a Sanger sequencing result of the library sequence obtained by assembling a degenerate sticky end, which proves that the desired gene mutation library is obtained.

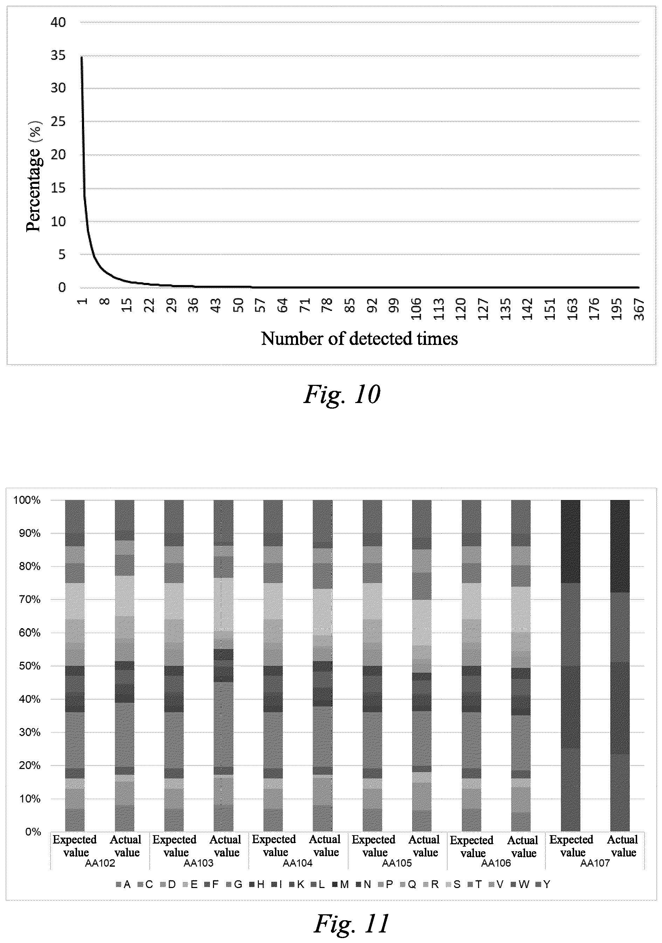

Fig. 10 is a sequence distribution diagram of NGS sequencing of the library sequence obtained by assembling a degenerate sticky end, and Table 2 is a sequence distribution table of NGS sequencing of the library sequence obtained by assembling a degenerate sticky end.

**Table 4. Sequence distribution table, by NGS sequencing, of the library sequence obtained by assembling a degenerate sticky end.**

| Number of detected times of sequences | % of total number of sequences | Count |
|---|---|---|
| 1 | 34.72731754 | 620006 |
| 2 | 13.83322308 | 246972 |
| 3 | 8.600282761 | 153546 |
| 4 | 6.152197881 | 109839 |
| 5 | 4.710222188 | 84094 |
| 6 | 3.759990661 | 67129 |
| 7 | 3.080016084 | 54989 |
| 8 | 2.578414205 | 46034 |
| 9 | 2.189449654 | 39089 |
| 10 | 1.88074763 | 33578 |
| 11 | 1.631243758 | 29123 |
| 12 | 1.425476997 | 25450 |
| 13 | 1.258155311 | 22463 |
| 14 | 1.114544016 | 19899 |
| 15 | 0.996043945 | 17783 |
| 16 | 0.891604083 | 15918 |
| 17 | 0.800186778 | 14286 |
| 18 | 0.721688263 | 12885 |
| 19 | 0.656627366 | 11723 |
| 20 | 0.598414984 | 10684 |
| 21 | 0.544820162 | 9727 |
| 22 | 0.498073855 | 8892 |
| 23 | 0.457605354 | 8170 |
| 24 | 0.420768642 | 7512 |
| 25 | 0.388082545 | 6929 |
| 26 | 0.356901047 | 6372 |
| 27 | 0.330337108 | 5898 |
| 28 | 0.308183198 | 5502 |
| 29 | 0.286963176 | 5123 |
| 30 | 0.264134791 | 4716 |
| 31 | 0.245197608 | 4378 |
| 32 | 0.229736566 | 4102 |
| 33 | 0.215053763 | 3839 |
| 34 | 0.200319079 | 3576 |
| 35 | 0.188282294 | 3362 |
| 36 | 0.175363503 | 3131 |
| 37 | 0.162963539 | 2909 |
| 38 | 0.152690766 | 2726 |
| 39 | 0.143196233 | 2557 |
| 40 | 0.135569477 | 2420 |
| >41 | 2.689910109 | 48024 |

Fig. 11 is an amino acid distribution of NGS sequencing of the library sequence obtained by assembling a degenerate sticky end. It can be seen from the above data, the distribution of the location, type and frequency of amino acid mutations in the library sequence is fully consistent with the expected design.

It should also be noted that, on the premise that it can be implemented and does not obviously violate the gist of the present invention, any technical feature or combination of technical features described as a constituent part of a technical solution in the present description can also be applied to other technical solutions; in addition, on the premise that it can be implemented and does not obviously violate the gist of the present invention, the technical features described as constituent parts of different technical solutions can also be combined in any way to form other technical solutions. The present invention also comprises technical solutions obtained by combining in the above-mentioned cases, and these technical solutions are equivalent to being described in the present invention.

The present invention was described above by specific embodiments and examples, but those skilled in the art should understand that these are not intended to limit the scope of the present invention. The scope of the present invention should be determined by the claims.

### Industrial Applicability

The present invention provides a large-storage capacity library construction method, capable of synthesizing relatively few oligomer sequences, then assembling same to become a large-storage capacity library, which greatly saves the cost for large-capacity library construction and solves the problem of expensive synthesis of the large-storage capacity library.

## Claims

1. A method for constructing a large-storage capacity gene mutation library, **characterized in that** the method comprises:
(1) designing and synthesizing two or more oligomer pools having a mutant nucleotide and a restriction site of a restriction endonuclease according to a nucleotide sequence encoding an amino acid sequence which requires library construction, wherein after digested, two adjacent oligomer pools designed produce same sticky ends;
(2) amplifying the oligomer pools;
(3) assembling the oligomer pools in a reaction system to obtain an assembled oligomer pool;
and (4) amplifying the assembled oligomer pool to obtain the large-storage capacity gene mutation library.

2. The method according to claim 1, **characterized in that** step (2) comprises: performing PCR amplification on each oligomer pool using a high-fidelity DNA polymerase by taking each oligomer pool as a template and taking the forward primer and reverse primer designed according to the sequence of each oligomer pool as a primer pair, to obtain each amplified oligomer pool.

3. The method according to claim 1 or 2, **characterized in that** step (3) comprises: adding each amplified oligomer pool, simultaneously adding the restriction endonuclease and a DNA ligase, and assembling each amplified oligomer pool by using a restriction-ligation method to obtain the assembled oligomer pool.

4. The method according to any one of claims 1-3, **characterized in that** step (4) comprises: performing PCR amplification on the assembled oligomer pool using the high-fidelity DNA polymerase by taking the assembled oligomer pool as a template and taking a forward primer of a first oligomer pool and a reverse primer of a last oligomer pool as a primer pair, to obtain the large-storage capacity gene mutation library.

5. The method according to any one of claims 1-4, **characterized in that** the storage capacity of the gene mutation library is up to 10⁵, preferably up to 10⁶, more preferably up to 10⁷, more preferably up to 10⁸, more preferably up to 10⁹, and more preferably up to 10¹⁰.

6. The method according to any one of claims 1-5, **characterized in that** the restriction endonuclease is a type IIS restriction endonuclease.

7. The method according to claim 6, **characterized in that** the type IIS restriction endonuclease is selected from one or more of *Acu*I*, Alw*I*, Bbs*I*, Bbv*I*, Bcc*I*, Bce*AI*, Bci*VI*, BfuAI, Bmr*I*, Bpm*I*, Bpu*EI*, Bsa*I*, Bse*RI*, Bsg*I*, Bsm*AI*, Bsm*BI*, Bsm*FI*, BspMl, Bsp*QI*, Bsr*DI*, Btg*ZI*, Bts*CI*, Bts*I*, Ear*I*, Eci*I*, Eco*P15I*, Fau*I*, Fok*I*, Hga*I*, Hph*I*, HpyAV, Mbo*II*, Mme*I*, Mnl*I*, Ple*I*, Sap*I and *Sfa*NI*.*

8. The method according to claim 7, **characterized in that** the type IIS restriction endonuclease is *Bsa*I*.*

9. The method according to any one of claims 1-8, **characterized in that** step (1) comprises:
(i) identifying the sticky ends in the coding nucleotide sequence, and dividing the sequence into two or more fragments corresponding to the two or more oligomer pools according to the 3' ends of the sticky ends,
(ii) if the sequence is divided into two fragments, then
sequentially introducing a reverse complement sequence of a recognition sequence of the restriction endonuclease and a specific sequence 1 at 3' end after a sticky end of a first fragment, to obtain an oligomer pool 1, and sequentially introducing a sticky end, a recognition sequence of the restriction endonuclease and a specific sequence 2 at 5' end of a second fragment, to obtain an oligomer pool 2;
or if the sequence is divided into n fragments, wherein n is a positive integer greater than or equal to 3, then
sequentially introducing a reverse complement sequence of a recognition sequence of the restriction endonuclease and a specific sequence 1 at 3' end after a sticky end of a first fragment, to obtain an oligomer pool 1; and sequentially introducing a sticky end, a recognition sequence of the restriction endonuclease and a specific sequence 2 at 5' end of a second fragment, sequentially introducing a reverse complement sequence of a recognition sequence of the restriction endonuclease and a specific sequence 3 at 3' end after a sticky end of a second fragment, to obtain an oligomer pool 2; and so on, sequentially introducing a sticky end, a recognition sequence of the restriction endonuclease and a specific sequence 2n-2 at 5' end of a n^{th} fragment and sequentially introducing a reverse complement sequence of a recognition sequence of the restriction endonuclease and a specific sequence 2n-1 at 3' end after a sticky end of a n^{th} fragment, to obtain an oligomer pool n.

10. The method according to claim 9, **characterized in that** the specific sequence 1-the specific sequence 2n-1 are random sequences that are not homologous to the original coding nucleotide sequence.

11. The method according to any one of claims 6-10, **characterized in that** the sticky end is a single sticky end or a degenerate sticky end.

12. The method according to claim 11, **characterized in that** the sticky end is a single sticky end, and the number of the oligomer pools is 2-6, preferably 2.

13. The method according to claim 11, **characterized in that** the sticky end is a degenerate sticky end, and the number of the oligomer pools is 2.

14. The method according to any one of claims 6-13, **characterized in that** the GC content of the sticky end is 50%-75%.

15. The method according to any one of claims 6-14, **characterized in that** the sticky end does not contain a palindromic structure.

16. The method according to any one of claims 2-15, **characterized in that** the restriction-ligation method in step (3) is Golden Gate cloning.

17. The method according to any one of claims 2-16, **characterized in that** the PCR amplification system in step (2) further comprises bovine serum albumin.

18. The method according to any of the claims 1-17, **characterized in that** the method further comprises
(5) recovering and/or purifying the product of the gene mutation library obtained in step (4), to obtain a final library product.

19. The method according to claim 18, **characterized in that** the product of the gene mutation library obtained in step (4) is recovered and/or purified by gel electrophoresis.

20. The method according to claim 18 or 19, **characterized in that** the method further comprises
(6) sequencing the final library product obtained in step (5) to verify sequence distribution and/or detect amino acid distribution.

21. The method according to claim 20, **characterized in that** the sequencing is Sanger sequencing and/or NGS sequencing.

22. The method according to any one of claims 1-21, **characterized in that** the number of the mutant nucleotide in each oligomer pool synthesized in step (1) is 1-108, preferably 1-21.

23. The method according to any one of claims 1-22, **characterized in that** the mutant nucleotide in each oligomer pool synthesized in step (1) encodes a mutation of 1-36, preferably 1-7 amino acids.

24. The method according to any one of claims 1-23, **characterized in that** the mutant amino acids encoded by the mutant nucleotide in each oligomer pool synthesized in step (1) are either adjacent or non-adjacent.

25. The method according to any one of claims 2-24, **characterized in that** the high-fidelity DNA polymerase is selected from one or more of Phusion DNA polymerase, Q5 polymerase and primerSTAR polymerase.

26. A gene mutation library constructed by the method according to any one of claims 1-25.

27. Use of the gene mutation library constructed by the method according to any one of claims 1-25 for screening proteins or polypeptides.

28. A method for analyzing the relationship between an amino acid mutation in a protein and the property, regulation and/or function of the protein, **characterized in that** the method comprises the following steps:
(1) constructing a gene mutation library by using the method according to any one of claims 1-25;
(2) comparing the property, regulation and/or function of the protein encoded by a mutant gene in the constructed gene mutation library with that of a unmutated protein; and
(3) analyzing the relationship between the amino acid mutation in the protein and the property, regulation and/or function of the protein.


Sequence Listing

<110>  Nanjing GenScript Biotech Co., Ltd.

<120>  METHOD FOR CONSTRUCTING A GENE MUTATION LIBRARY

<130>  1

<150>  CN201911316464.2
<151>  2019-12-19

<160>  27

<170>  PatentIn version 3.5

<210>  1
<211>  40
<212>  PRT
<213>  Artificial sequence

<220>
<223>  Amino acid sequence which requires library construction


<220>
<221>  Variant
<222>  (8)..(8)
<223>  4 of the 20 amino acids

<220>
<221>  Variant
<222>  (9)..(9)
<223>  16 of the 20 amino acids

<220>
<221>  Variant
<222>  (10)..(10)
<223>  16 of the 20 amino acids

<220>
<221>  Variant
<222>  (12)..(12)
<223>  16 of the 20 amino acids

<220>
<221>  Variant
<222>  (20)..(20)
<223>  16 of the 20 amino acids

<220>
<221>  Variant
<222>  (21)..(21)
<223>  16 of the 20 amino acids

<220>
<221>  Variant
<222>  (33)..(33)
<223>  3 of the 20 amino acids

<400>  1

Arg Pro Asp Val Asn Ala Ser Xaa Xaa Xaa Gly Xaa Thr Pro Leu His
1               5                   10                  15

Leu Ala Ala Xaa Xaa Gly His Leu Glu Ile Val Glu Val Leu Leu Lys
            20                  25                  30

Xaa Gly Ala Asp Val Arg Pro Asp
        35                  40

<210>  2
<211>  120
<212>  DNA
<213>  Artificial sequence

<220>
<223>  Nucleotide sequence encoding amino acid sequence 1

<220>
<221>  Misc_feature
<222>  (22)..(30)
<223>  n is a, c, g or t

<220>
<221>  Misc_feature
<222>  (34)..(36)
<223>  n is a, c, g or t

<220>
<221>  Misc_feature
<222>  (58)..(63)
<223>  n is a, c, g or t

<220>
<221>  Misc_feature
<222>  (97)..(99)
<223>  n is a, c, g or t

<400>  2
agacctgacg ttaacgctag cnnnnnnnnn ggtnnnactc cgctgcacct ggctgctnnn      60

nnnggtcacc tggaaatcgt tgaagttctg ctgaagnnng gtgctgacgt gagacctgac      120

<210>  3
<211>  51
<212>  DNA
<213>  Artificial sequence

<220>
<223>  First oligomer

<220>
<221>  Misc_feature
<222>  (22)..(30)
<223>  n is a, c, g or t

<220>
<221>  Misc_feature
<222>  (34)..(36)
<223>  n is a, c, g or t

<400>  3
agacctgacg ttaacgctag cnnnnnnnnn ggtnnnactc cgctgcacct g                51


<210>  4
<211>  6
<212>  DNA
<213>  Artificial sequence

<220>
<223>  Reverse complement sequence of the recognition sequence of BsaI

<400>  4
gagacc                                                                   6


<210>  5
<211>  58
<212>  DNA
<213>  Artificial sequence

<220>
<223>  The sequence with the restriction enzyme site introduced


<220>
<221>  Misc_feature
<222>  (22)..(30)
<223>  n is a, c, g or t

<220>
<221>  Misc_feature
<222>  (34)..(36)
<223>  n is a, c, g or t

<400>  5
agacctgacg ttaacgctag cnnnnnnnnn ggtnnnactc cgctgcacct gagagacc         58


<210>  6
<211>  20
<212>  DNA
<213>  Artificial sequence

<220>
<223>  Specific sequence

<400>  6
acctacgtcc aaatcggctg                                                   20


<210>  7
<211>  78
<212>  DNA
<213>  Artificial sequence

<220>
<223>  Oligomer pool-1


<220>
<221>  Misc_feature
<222>  (22)..(30)
<223>  n is a, c, g or t

<220>
<221>  Misc_feature
<222>  (34)..(36)
<223>  n is a, c, g or t

<400>  7
agacctgacg ttaacgctag cnnnnnnnnn ggtnnnactc cgctgcacct gagagaccac      60

ctacgtccaa atcggctg                                                    78


<210>  8
<211>  69
<212>  DNA
<213>  Artificial sequence

<220>
<223>  Specific sequence of the second oligomer


<220>
<221>  Misc_feature
<222>  (7)..(12)
<223>  n is a, c, g or t

<220>
<221>  Misc_feature
<222>  (46)..(48)
<223>  n is a, c, g or t

<400>  8
gctgctnnnn nnggtcacct ggaaatcgtt gaagttctgc tgaagnnngg tgctgacgtg      60

agacctgac                                                              69


<210>  9
<211>  6
<212>  DNA
<213>  Artificial sequence

<220>
<223>  Recognition sequence of restriction endonuclease BsaI

<400>  9
ggtctc                                                                  6


<210>  10
<211>  80
<212>  DNA
<213>  Artificial sequence

<220>
<223>  The sequence obtained by additionally adding a base G

<220>
<221>  Misc_feature
<222>  (18)..(23)
<223>  n is a, c, g or t

<220>
<221>  Misc_feature
<222>  (57)..(59)
<223>  n is a, c, g or t

<400>  10
ggtctcgcct ggctgctnnn nnnggtcacc tggaaatcgt tgaagttctg ctgaagnnng        60

gtgctgacgt gagacctgac                                                    80

<210>  11
<211>  21
<212>  DNA
<213>  Artificial sequence

<220>
<223>  Specific sequence

<400>  11
actcctaagt aaaatgtgca c                                                  21

<210>  12
<211>  101
<212>  DNA
<213>  Artificial sequence

<220>
<223>  Oligomer pool-2

<220>
<221>  Misc_feature
<222>  (39)..(44)
<223>  n is a, c, g or t

<220>
<221>  Misc_feature
<222>  (78)..(80)
<223>  n is a, c, g or t

<400>  12
actcctaagt aaaatgtgca cggtctcgcc tggctgctnn nnnnggtcac ctggaaatcg        60

ttgaagttct gctgaagnnn ggtgctgacg tgagacctga c                           101

<210>  13
<211>  20
<212>  DNA

<213>  Artificial sequence

<220>
<223>  1F

<400>  13
agacctgacg ttaacgctag                                                    20


<210>  14
<211>  20
<212>  DNA
<213>  Artificial sequence

<220>
<223>  1R

<400>  14
cagccgattt ggacgtaggt                                                    20


<210>  15
<211>  20
<212>  DNA
<213>  Artificial sequence

<220>
<223>  2F

<400>  15
actcctaagt aaaatgtgca                                                    20


<210>  16
<211>  20
<212>  DNA
<213>  Artificial sequence

<220>
<223>  2R

<400>  16
gtcaggtctc acgtcagcac                                                    20


<210>  17
<211>  120
<212>  DNA
<213>  Artificial sequence

<220>
<223>  Sequence of assembled oligomer pool


<220>
<221>  Misc_feature
<222>  (22)..(30)
<223>  n is a, c, g or t

<220>
<221>  Misc_feature

<222>  (34)..(36)
<223>  n is a, c, g or t

<220>
<221>  Misc_feature
<222>  (58)..(63)
<223>  n is a, c, g or t

<220>
<221>  Misc_feature
<222>  (97)..(99)
<223>  n is a, c, g or t

<400>  17
agacctgacg ttaacgctag cnnnnnnnnn ggtnnnactc cgctgcacct ggctgctnnn      60

nnnggtcacc tggaaatcgt tgaagttctg ctgaagnnng gtgctgacgt gagacctgac      120


<210>  18
<211>  32
<212>  PRT
<213>  Artificial sequence

<220>
<223>  Amino acid sequence which requires library construction


<220>
<221>  Variant
<222>  (14)..(14)
<223>  18 of the 20 amino acids

<220>
<221>  Variant
<222>  (15)..(15)
<223>  18 of the 20 amino acids

<220>
<221>  Variant
<222>  (16)..(16)
<223>  18 of the 20 amino acids

<220>
<221>  Variant
<222>  (17)..(17)
<223>  18 of the 20 amino acids

<220>
<221>  Variant
<222>  (18)..(18)
<223>  18 of the 20 amino acids

<220>
<221>  Variant
<222>  (19)..(19)
<223>  18 of the 20 amino acids

<220>
<221>  Variant
<222>  (19)..(19)

<223>  4 of the 20 amino acids

<400>  18

Lys Thr Glu Asp Thr Ala Val Tyr Tyr Cys Ser Arg Asp Xaa Xaa Xaa
1               5                   10                  15

Xaa Xaa Xaa Asp Ala Trp Gly Gln Gly Thr Leu Val Thr Val Ser Ser
            20                  25                  30

<210>  19
<211>  96
<212>  DNA
<213>  Artificial sequence

<220>
<223>  Nucleotide sequence encoding amino acid sequence 18

<220>
<221>  Misc_feature
<222>  (40)..(57)
<223>  n is a, c, g or t

<400>  19
aaaaccgagg acacggccgt gtattactgt agtagagatn nnnnnnnnnn nnnnnnngac      60

gcctggggcc aaggaaccct ggtcaccgtc tcctcg                                96

<210>  20
<211>  51
<212>  DNA
<213>  Artificial sequence

<220>
<223>  First oligomer

<220>
<221>  Misc_feature
<222>  (40)..(51)
<223>  n is a, c, g or t

<400>  20
aaaaccgagg acacggccgt gtattactgt agtagagatn nnnnnnnnnn n               51

<210>  21
<211>  58
<212>  DNA
<213>  Artificial sequence

<220>
<223>  The sequence of the first oligomer  with the restriction enzyme site
introduced

<220>

<221>  Misc_feature
<222>  (40)..(51)
<223>  n is a, c, g or t

<400>  21
aaaaccgagg acacggccgt gtattactgt agtagagatn nnnnnnnnnn nagagacc          58


<210>  22
<211>  78
<212>  DNA
<213>  Artificial sequence

<220>
<223>  Oligomer pool-1


<220>
<221>  Misc_feature
<222>  (40)..(51)
<223>  n is a, c, g or t

<400>  22
aaaaccgagg acacggccgt gtattactgt agtagagatn nnnnnnnnnn nagagaccac          60

ctacgtccaa atcggctg                                                      78


<210>  23
<211>  51
<212>  DNA
<213>  Artificial sequence

<220>
<223>  Second oligomer


<220>
<221>  Misc_feature
<222>  (1)..(12)
<223>  n is a, c, g or t

<400>  23
nnnnnnnnnn nngacgcctg gggccaagga accctggtca ccgtctcctc g                 51


<210>  24
<211>  56
<212>  DNA
<213>  Artificial sequence

<220>
<223>  The sequence obtained by additionally adding a base G


<220>
<221>  Misc_feature
<222>  (8)..(17)
<223>  n is a, c, g or t

<400>  24

ggtctcgnnn nnnnnnngac gcctggggcc aaggaaccct ggtcaccgtc tcctcg          56

<210>  25
<211>  77
<212>  DNA
<213>  Artificial sequence

<220>
<223>  The final sequence of the second oligomer

<220>
<221>  Misc_feature
<222>  (29)..(38)
<223>  n is a, c, g or t

<400>  25
actcctaagt aaaatgtgca cggtctcgnn nnnnnnnnga cgcctggggc caaggaaccc          60

tggtcaccgt ctcctcg                                                          77

<210>  26
<211>  20
<212>  DNA
<213>  Artificial sequence

<220>
<223>  1sF

<400>  26
aaaaccgagg acacggccgt                                                       20

<210>  27
<211>  20
<212>  DNA
<213>  Artificial sequence

<220>
<223>  2sR

<400>  27
cgaggagacg gtgaccaggg                                                       20